(19) Europäisches Patentamt / European Patent Office / Office européen des brevets

(11) EP 2 006 684 B1

(12) **EUROPEAN PATENT SPECIFICATION**

(45) Date of publication and mention of the grant of the patent:
**18.04.2012 Bulletin 2012/16**

(21) Application number: **06833308.7**

(22) Date of filing: **24.11.2006**

(51) Int Cl.:
***G01N 33/543*** *(2006.01)* ***B05D 1/20*** *(2006.01)*

(86) International application number:
**PCT/JP2006/323504**

(87) International publication number:
**WO 2007/108173 (27.09.2007 Gazette 2007/39)**

(54) **BIOMOLECULAR ELEMENT, MULTILAYER MOLECULAR THIN FILM OF FATTY ACID AND LIPID TO BE SUPPORT FOR BIOSENSOR MOLECULE AND METHOD FOR PRODUCING THE SAME**

BIOMOLEKÜLELEMENT, MOLEKULARER DÜNNER MEHRSCHICHTENFILM AUS FETTSÄURE UND LIPID ALS TRÄGER FÜR EIN BIOSENSORMOLEKÜL SOWIE VERFAHREN ZUR HERSTELLUNG DAVON

ELÉMENT BIOMOLÉCULAIRE, FILM MOLÉCULAIRE MINCE MULTICOUCHE D'ACIDE GRAS ET DE LIPIDE SERVANT DE SUPPORT POUR UNE MOLÉCULE DE BIOCAPTEUR, ET LEUR PROCÉDÉ DE PRODUCTION

(84) Designated Contracting States:
**CH DE FR GB LI**

(30) Priority: **23.03.2006 JP 2006080442**

(43) Date of publication of application:
**24.12.2008 Bulletin 2008/52**

(73) Proprietor: **National University Corp. Akita Univ.**
**1-1 Tegata-Gakuen-machi**
**Akita-shi, Akita 010-8502 (JP)**

(72) Inventor: **TSUJIUCHI, Yutaka**
**Akita-shi, Akita 010-8502 (JP)**

(74) Representative: **TBK**
**Bavariaring 4-6**
**80336 München (DE)**

(56) References cited:
**JP-A- 2003 511 679 JP-A- 2005 172 522**

- **HOWARTH VA, PETTY MC, ANCELIN H, YARWOOD J: "structural characterisation of phosphoplipid Langmuir-Blodgett multilayers containing valinamycin" VIBRATIONAL SPECTROSCOPY, vol. 1, 1990, pages 29-33, XP002518415**
- **BRUCKNER-LEA C, PETELENZ D, JANATA J: "use of Poly(octadec-1-ene-maleic anhydride) for interfacing bilayer membranes to solid supports in sensor applications" MIKROCHEMICA ACTA, vol. 1, 1990, pages 169-185, XP002518416**
- **PETERSON IR: "Langmuir-Blodgett films" J. PHYS. D: APPL. PHYS., vol. 23, 1990, pages 379-395, XP002518417**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

EP 2 006 684 B1

## Description

Technical Field

The present invention relates to a thin film for realizing, in an artificial thin film system, functional expression of a membrane protein, which reacts specifically to a ion, molecule, or any of other various chemical species, or a membrane protein, which functions in a specific photoreaction or light driven ion pump, etc., and to a method for producing the organic thin film.

Background Art

Living organisms act upon sensing an endless variety of substances that are present in an external environment on a molecular level. These advanced functions begin with a physicochemical reaction process occurring at a membrane protein in contact with the external environment. Although such a reaction process can be utilized if it can be connected to an interface of an artificial system, this is not easy because functional expression of a membrane protein largely involves a molecular environment of other molecules surrounding the molecule.
Lipids are principally present in a molecular environment related to functional expression of a membrane protein.
As can be seen with phosphatidylcholine, a lipid has a molecular structure that is divided into two hydrophobic hydrocarbon chains and a hydrophilic phosphate group, and such molecules are paired so as to face each other upside down and envelop a hydrophobic region of a single membrane protein molecule with the hydrocarbon chains, and the membrane protein is thereby surrounded by a large number of several dozen to a hundred lipid molecules and functions in a membrane spatial structure, called a lipid bilayer, that is seen in cell membranes.
This property, of stabilizing as a capsule-like structure by a self-organizing ability unique to lipid molecules and even becoming a spherical structure called a liposome, is a cause of difficulty in fabricating a planar artificial film.
Conventionally, various measures have been taken toward solving this problem. In particular, in the 1980s, Ikonen et al attempted film fabrication by a Langmuir-Blodgett method of spreading a hexane mixture solution of a purple membrane, containing a membrane protein bacteriorhodopsin, which is a light driven proton pump, and a phospholipid soya PC (phosphatidylcholine contained in soya beans), on an aqueous solution and then transferring onto a substrate (see Non-Patent Document).
Although various attempts have been made conventionally toward fabricating a lipid film by applying the Langmuir-Blodgett method described above, a significant advance has not been made toward achieving the theme of forming a planar artificial film of a lipid. Even with the method of Ikonen et al, the film structure is low in uniformity and a purple membrane, formed by no less than a hundred times of accumulation, has only been used, for example, in research of photoreaction characteristics.
An optical memory, can be considered as an application of making a molecule be present in a film structure formed by such accumulation and function as a molecule of specific photoreaction. As long as the film structure is a closed system, the film is independent and not suited for continuous substance transport or energy conversion with respect to another device. Although a planar film of lipid molecules that is fabricated by taking advantage of a process of swelling by and evaporation of a solvent is not a closed system but is an open system, the film structure, maintained by lateral binding of the lipid molecules due to a hydrophobic interaction of the molecules, has a problem in terms of strength and is unsuited for a device for substance transport that is continuous in both time and space.
There are difficulties in terms of structure, strength, in introducing a biomolecular element having a function of ion transport, or a biosensor molecule into such a film structure fabricated by a conventional method.
A thin film, having both strength and flexible structural characteristics, enabling formation of a planar structure of lipid molecules and continuous transport of ionic substances at the same time, and a method for fabricating such a thin film must thus be devised.
Among recent new methods besides the above, there is a method, developed by Tamiya et al, of forming a planar film of a lipid by covering micropores. With this method, lipid molecules or other molecules are dissolved in an organic solvent and supplied as a thin film forming material to a substrate in which small pores are formed, and swelling by and evaporation of the solvent is taken advantage of to obtain a planar film of the lipid molecules (see Patent Document 1). This method is characteristic in that a planar film can be fabricated accurately under fixed conditions.
[Patent Document 1] Japanese Published Unexamined Patent Application No. 2005-245331
[Non-Patent Document 1] Marjo Ikonen, Jouko Peltonen, Elina Vuorimaa, and Helge Lemmetyinen: "Study of photocycle and spectral properties of bacteriorhodopsin in Langmuir-Blodgett films." Thin Solid Films 213 (1992) 277-284.
HOWARTH VA, PETTY MC, ANCELIN H, YARWOOD J: "structural characterisation of phospholipid Langmuir-Blodgett multilayers containing valinamycin" VIBRATIONAL SPECTROSCOPY, vol. 1, 1990, pages 29-33, is a scientific publication about the structural characterization of phospholipid Langmuir-Blodgett (LB) multilayers containing valinomycin.

BRUCKNER-LEA C, PETELENZ D, JANATA J: "Use of Poly(octadec-1-ene-maleic anhydride) for interfacing bilayer membranes to solid supports in sensor applications" MIKROCHEMICA ACTA, vol. 1, 1990, pages 169-185, is directed to the use of poly(octadec-1-ene-maleic anhydride) for interfacing bilayer membranes on solid supports.

Disclosure of the Invention

Object(s) to be Solved by the Invention

The present invention engages the difficult problems of the conventional methods and an object thereof is to provide a flexible organic thin film of high strength, to be a support for a membrane protein or other molecule that functions as a biomolecular element or biosensor and enabling the molecule to function in the same manner as in a living body, and a method for producing the organic thin film.

Means for Solving the Problem

A thin film to be a support for a membrane protein or other biomolecular element or biosensor molecule according to the present invention comprises a laminate of molecular thin films of a fatty acid and a lipid; wherein the laminate of molecular thin films comprises a first type layer of a fatty acid and a second type layer of a lipid; and the first type layer is formed on a substrate and the second type layer is joined onto the first type layer.

Preferred embodiments are depicted in the dependent claims.

The fatty acid may be stearic acid or other alkane acid.

The fatty acid may be retinoic acid or other fatty acid having an ionone ring.

The fatty acid is preferably a combination of a film portion that is stearic acid or other alkane acid and a film portion that is retinoic acid or other fatty acid having an ionone ring.

The lipid is preferably a phospholipide. The phospholipid is preferably dimyristoylphosphatidylcholine, dipalmitoylphosphatidylcholine.

Preferably, the first type layer of the thin film is a three-layer molecular thin film of a fatty acid.

It is further preferred that the second type layer of the thin film is a two-layer molecular thin film of a lipid.

Further, the surface of the thin film is preferably constituted of a collection of uniform, curved protruding surfaces of approximately 200 nm.

In the thin film a membrane protein is preferably stabilized and expresses a function in the two-layer molecular thin films of a lipid.

In a method for producing an organic thin film according to the present invention a first layer of fatty acid is formed on a substrate by a vertical dipping method and a second type of layer of a lipid suspended in a solvent is dispersed by ultrasonic wave application is joined from above to the first layer of fatty acid.

The organic thin film may have a basic configuration of having the lipid molecules on an uppermost stage and the fatty acid below.

An organic solvent is preferably used as the solvent in which the lipid is dispersed before the organic thin film joining process.

An organic solvent with a boiling point in a range of 40°C to 70°C is preferably used as the organic solvent.

Hexane is preferably used as the organic solvent.

To introduce a membrane protein or other molecule in the organic thin film, the membrane protein or other molecule is preferably mixed in a phospholipid suspended in the organic solvent to obtain the organic thin film having the lipid molecules and the membrane protein or other molecule at the uppermost stage and the fatty acid below.

In the vertical dipping method, the organic thin film is preferably formed accumulatingly on the substrate by a Langmuir-Blodgett method.

Effect(s) of the Invention

With the present invention, an organic thin film to be a support for a biomolecular element or a biosensor molecule is fabricated by forming a second type layer of lipid molecules above a layer of fatty acid molecules that is a first type thin film layer, formed on an inorganic substrate. By selection and combination of fatty acid molecules, a thin film structure with a characteristic pattern structure can be obtained.

By the present invention of the characteristic film structure, when a membrane protein functioning as a receptor, or an ion transporting membrane protein can be introduced in a lipid film with stability and can be made to express a physiological function, application to a device for detecting external molecules and chemical species, application to devices for ion transport from an external environment into an apparatus system, can be anticipated.

Best Mode(s) for Carrying Out the Invention

Upon carrying out detailed analysis of surface structures of inorganic compounds, the present inventors have attempted accumulation of lipid molecules by an operation of joining and layering on a surface of an inorganic compound. However, as a result of analyzing forms of assembly of lipid molecules, the present inventors arrived at a concept that, for forming a uniform thin film of lip id molecules, it is difficult to perform direct accumulation onto a smooth inorganic compound, such as a silicon dioxide (SiO2) crystal plate or a glass substrate. It is considered that this is because lipid molecules are weak in adsorptivity to inorganic substrates and have a property of not forming a uniform, closest-packed bilayer film.

Upon conducting a review of method, the present inventors arrived at an idea that a method of joining organic molecules of high surface activity that can form a uniform closest-packed bilayer film to an inorganic substrate in advance and forming a film of lipid molecules above the organic molecules may be effective.

Fatty acid molecules include many molecules of high surface activity. A first type layer can be formed on an inorganic substrate using fatty acid molecules. In a case where a lipid is to be joined as a second type layer onto the fatty acid molecules, because the lipid does not directly contact a silicon dioxide (SiO2) crystal plate that is the substrate, a large disturbance of film properties does not occur. Such a difference is considered to arise due to there being a large difference in a so-called attachment phenomenon (difference in wetting properties). On a film of the fatty acid, the lipid molecules form, in accordance with the phenomenon of attachment with the fatty acid, a filmof high density that cannot be achieved on the inorganic substrate. Also, in accordance with the type of fatty acid used, different characteristics arise in the structure of the film formed. By combining these, possibilities of film structure design are widened.

An organic thin film to be a support for a biomolecular element or biosensor molecular and a method for manufacturing the same according to the present invention shall now be described in detail.

Proteins can have various properties according to differences in structure, such as an alignment of amino acids, which are the structural units. Many proteins have a property of sensitively reacting to temperature, light, or other environmental change. Recently, in particular, research and development are being carried out in various fields on selective permeation functions of membrane proteins present in biological membranes. Forming of a compartment by partitioning and permeation of specific substances lead to functions such as condensation, isolation, of a specific substance in the compartment.

To sense, recognize, or receive a physical state change or substance accompanying information, a receptor protein of a membrane receives an information transmitting substance from an exterior, or circumstances of an adjacent cell is recognized by a structure on an outer surface of a membrane. Functional expression is thereby triggered as a response to stimulus. The functions of biological membranes are extremely complex, diverse, and can be said to possess large possibilities. If these functions can be controlled by engineering, applications as energy-saving, resource-saving, environment-protecting process materials to devices, biosensors, bioelements, can be anticipated.

The functions of biological membranes are extremely complex, diverse, and differ significantly according to membrane system. The functions include compartment formation by partitioning, permeation and transport of specific substances. Starting with partitioning with respect to an external environment to form an internal environment called a cell, various compartments are formed by further partitioning within the cell. This leads to such functions as condensation, isolation, of a specific substance within a compartment. Meanwhile, regardless of such partitioning, only specific substances are made to pass through via small pores of a membrane or transported across a membrane based on actions of a transport protein inside the membrane. Functions, such as intake of necessary substances, discharge (secretion) of biosynthesized substances, discharge (excretion) of insoluble substances, maintenance of homeostasis inside the cell, are thereby performed. In regard to functions of recognizing and receiving information from the exterior, a messenger substance from the exterior is received by a receptor of the membrane or circumstances of an adjacent cell is recognized by a structure on the outer surface of the membrane. A functional expression is thereby triggered as a response to stimulus.

As a substance that supports such diverse functions of membrane proteins, a phospholipid in a biological membrane serves a role as a partition of the biological membrane and also serves an important role of stabilizing the membrane proteins and making the membrane proteins function as biomolecular elements.

Phosphatidylcholine, which is one type of phospholipid, is a representative glycerol lipid that is widely distributed among animals, plants, yeasts, and molds, constitutes cell membranes and other biological membranes as well as neurotransmitters in the brain and nerves, and is also contained in large amounts in the liver and is involved in metabolic activity.

Dimyristoylphosphatidylcholine (DMPC) is a molecule having a form with which an R group and an R' group of phosphatidylcholine is substituted by $CH_3(CH_2)_{12}$ group of myristic acid ($CH_3(CH_2)_{12}COOH$ group).

[Chemical Formula 1]

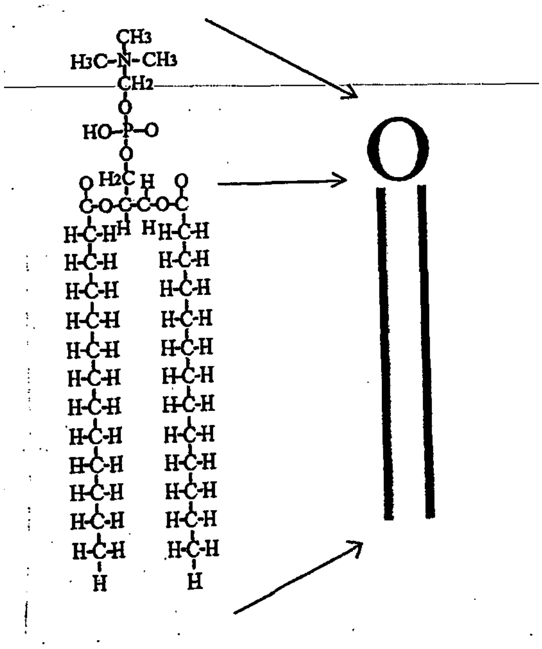

DMPC, the chemical structural formula of which is shown in Chemical Formula 1, is a saturated fatty acid with a molecular weight of 758.07. Structurally, this molecule has both a hydrophilic property and a hydrophobic property and thus exhibits surface activity and is suited for forming into a two-dimensional film. It is thus widely used as a principal component of artificial lipid films in research on biological membranes and reconstituted films. It is also included as a digestible surfactant in many food products and is used as a microencapsulating agent for drugs and low molecular weight compounds in medical and pharmaceutical fields.

When suspended in an aqueous solution, DMPC forms a lipid bilayer film and is a lipid that is useful for stabilization and functional expression of membrane proteins. However, due to this property of being insoluble in an aqueous solvent, DMPC is difficult to handle. An ability to freely and artificially form a lipid bilayer film is a major issue in seeking application methods of membrane proteins. According to research by Ikonen et al, it is reported that although it is difficult to form a film with a lipid being suspended in a hexane solvent as it is, by dispersing by ultrasonic waves and spreading on a water surface, accumulation onto a substrate by the Langmuir-Blodgett method or other film forming method becomes enabled.

Ikonen et al., performed an experiment of suspending bacteriorhodopsin (BR), which is an example of a membrane protein present in a purple membrane of an extreme halophile, a halobacterum halobium, and has a light-driven proton pump function, together with soya-PC, which is a phospholipid, and accumulating to no less than 100 layers on a substrate and analyzed photoreaction characteristics. However, with this example, each single layer of film is low in precision and a function as a precision biomolecular element is not achieved.

The present invention is arrived at by selecting DMPC for fabricating a highly precise two-dimensional lipid film, attempting fixation onto an inorganic substrate in a solitary form and lamination with another organic molecule, examining an accumulation state on the substrate, then using two different types of substances, that is, stearic acid and retinoic acid, which are fatty acids, to perform detailed examination of an interface state, with respect to DMPC that is accumulated above the fatty acids, and making careful examinations and comparisons toward a purpose of making DMPC molecules, which are lipid molecules, into a dense, two-dimensional form and thereby forming into a thin film. In making the careful examinations, the film structure is examined carefully by a scanning probe microscope and measuring and examining ultraviolet-visible absorption spectra and infrared absorption spectra.

[Chemical Formula 2]

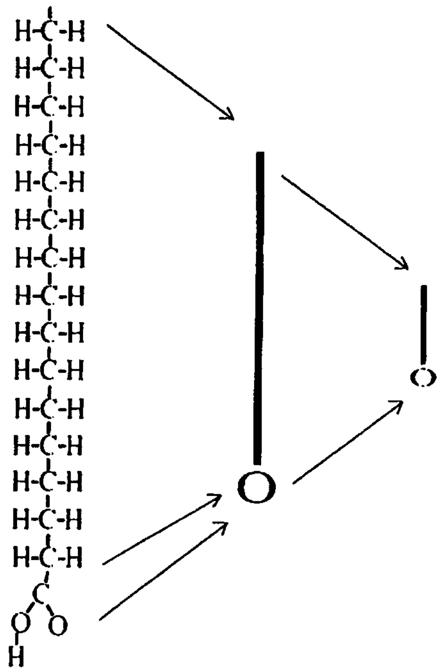

Stearic acid, which is one of the fatty acids used in the present invention, is the molecule expressed by Chemical Formula 2, and is a saturated, straight-chain fatty acid with the molecular formula $CH_3(CH_2)_{16}COOH$, a molecular weight of 284.48, and a melting point of 69. 6°C, and takes a form of white, leaf-like crystals. It is a component that is found most often as a natural saturated fatty acid and has a property of not becoming oxidized readily. In particular, it is contained in large amounts in beef fat, lard, soybean oil, and cacao butter. As applications, it is for example used in fatty acid soaps, surfactants, candles, abrasives. A lipid film formed for example by stearic acid and a phospholipid functions as a barrier. Because it not only isolates contents of a cell but also enables selective movement of ions and organic substances inside and outside the cell, stearic acid is suited for developing artificial devices by control of such properties. Because stearic acid structurally has a hydrophobic group and a hydrophilic group, it has surface activity and is thus suited for the Langmuir-Blodgett (LB) method, which is the film forming method used in the present invention, and can be formed as a stearic acid LB film on an inorganic substrate.

With the present invention, stearic acid molecules, which can be formed into a two-dimensional thin film, are accumulated in advance on an inorganic substrate and used for examination toward achieving thin film stabilization of DMPC molecules that form a lipid bilayer above the stearic acid molecules as shall be described below.

Retinoic acid, which is another fatty acid used for comparison with stearic acid in the present invention, shall now be described in an order starting from a related substance, retinal.

A retinoid molecule, called chromophore retinal, absorbs light energy inside the molecule of the membrane protein BR and is a chemical substance that is an aldehyde form of vitamin A. Retinal is bound to Lys216 of the protein via a protonated Schiff base linkage. When BR absorbs visible light, retinal undergoes structural isomerization from an all-trans form to a 13-cis form, and thereafter, BR changes in structure in accordance with the isomerized retinal to initiate a light-induced proton transport cycle. BR normally exhibits a purple color. This is due to BR having a light absorption band in the visible light region, and there is an absorption maximum at 568nm. Meanwhile, the all-trans form of retinal present in BR has an absorption maximum near 380nm. As a result of the retinal molecule binding to bacteriorhodopsin and interacting with amino acid residues inside the protein, the absorption shifts by nearly 190nm toward a longer wavelength side, and it is considered that the absorption maximumwavelength of BR consequently lies at 568nm. The chromophore retinal, which, in BR, is a prosthetic molecule that absorbs light energy and transmits the energy inside the protein, is extremely important as a molecule that serves as a starting point for attempting a biomimetic reproduction of expression of optical responsivity or other advanced function by a protein in a thin film or other artificial system.

However, retinal, which is thus an important organic molecule in terms of application to biodevices, is extremely unstable to light and denatures rapidly and is thus difficult to handle. Thus, retinoic acid, which is a related substance of retinal, is noted in the present invention.

[Chemical Formula 3]

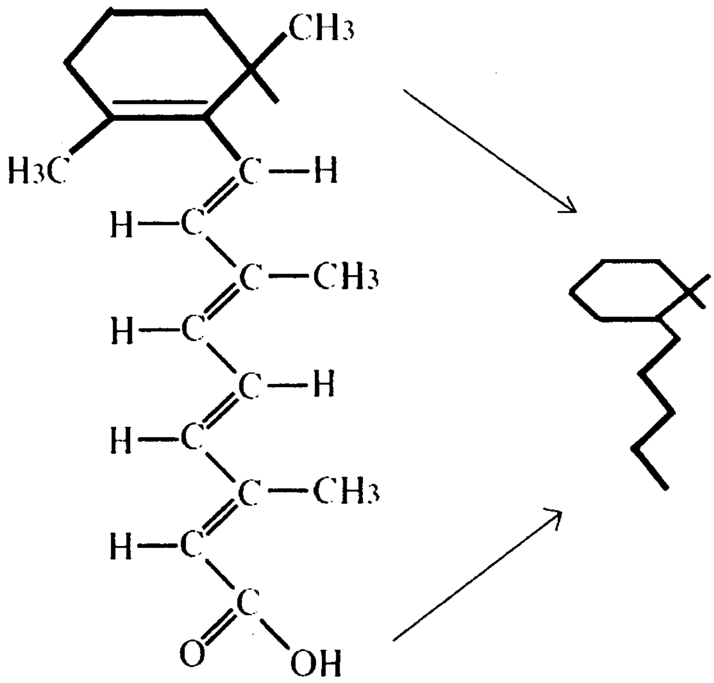

Retinoic acid is an organic molecule indicated by Chemical Formula 3 and has a molecular weight of 300.44, a slightly yellow color, and a melting point of 179 to 180°C. It has a beta ionone ring, exhibits a light absorption spectrum with an absorption maximum at a wavelength of 360nm, and has a carboxylic group terminal. There is only a slight difference with respect to retinal having the light absorption maximum wavelength of 380nm and the mechanisms by which optical isomerization occur are similar. The carboxylic group functions as a hydrophilic group, which, within the molecule, is most compatible with respect to water. Retinoic acid is thus stable in comparison to retinal, and because the hydrophilic region is large, it is suited for the LB method, which is the film forming method used in the present invention. It is thus suited as a film forming material substance for a biodevice.

Cavitation due to ultrasonic waves as a method for dispersing lipid molecules in an organic solvent, which is also used in the present invention, shall now be described briefly. Cavitation due to ultrasonic waves is widely used for purposes of organic compound synthesis, reaction, and reaction promotion, etc. Ultrasonic waves are especially effective for preparing various organic reagents. In the present invention, a table-top ultrasonic oscillator (NEOCLEANER-R, manufactured by Alex Corporation) is used to apply an ultrasonic treatment to a reagent to promote dispersion of an organic molecule in a solvent in a reagent preparation process.

The monolayer accumulation method used in the present invention shall now be described briefly. When a monolayer of organic molecules is formed on a water surface and the monolayer is successively transferred and layered on a solid surface, a film with a thickness of molecular order is formed. This method is also called the Langmuir-Blodgett method (LB method) after the names of the inventors, Langmuir and Blodgett, and is a widely-used, conventional method. The LB method, which is one type of interface adsorption method, is a film forming process that is normally carried out under normal temperature, normal pressure, and conditions close to thermal equilibrium and can be applied to various organic molecules. A range of dimensions of film-forming substance configurations spans several orders of magnitude from molecules, polymers, and spherical proteins of approximately nanometer order to molecular tissue fragments of micrometer order, such as the purple membrane that contains the BR protein.

One restriction of the LB method is that the film-forming substance must have surface activity. A generally seen characteristic of a surface active molecule is that the molecule has both a hydrophilic group and a hydrophobic group and, for example, with stearic acid, $CH_3(CH_2)_{16}COOH$, which is a typical surface active molecule, $CH_3(CH_2)_{16}$ is the hydrophobic group and -COOH is the hydrophilic group. The hydrophilic group has a tendency to dissolve in water, the hydrophobic group has a tendency to emerge into a gas phase, and thus the molecule as a whole exhibits a tendency to stay near the water surface. A surface active molecule, with which hydrophilicity and hydrophobicity are well balanced, becomes adsorbed onto a gas-water interface and forms a monolayer.

Although conventionally, film-forming molecules, such as stearic acid, that have a long-chain alkyl (with a

number of carbon atoms n of approximately 12 to 20) as a hydrophobic group, are thus used with the LB method in many cases, examples using film-forming substances without a long-chain alkyl and proteins and other substances, with which the distinction between the hydrophilic group and the hydrophobic group are not very clear, have been increasing recently. In principle, LB films that are formed by layering monolayers one by one have diverse possibilities in terms of composition and structure. Both stearic acid and retinoic acid, which are used in the present invention, are suited for the LB method, and DMPC can be suitable if it clears the conditions of dispersing as a monolayer on a water surface and becoming adsorbed as a surface active molecule by the balance of hydrophilicity and hydrophobicity.

(Principles of the LB Method)

In general, hydrophilic groups, which are portions that become intimate with water, tend to attract each other as portions that become intimate with water, and hydrophobic groups, which are portions that do not become intimate with water, tend to attract each other as portions that do not become intimate with water. In a vertical dipping method, which is generally used to fabricate an LB film, the above properties are made use of, and if a surface of a solid substrate becomes intimate with water, the solid substrate is immersed below the water surface in advance and after a monolayer film is fabricated on the water surface, the substrate is raised above the water surface. Because the water surface then swells slightly upward at a periphery of the solid substrate in accompaniment with the raising of the solid substrate, the hydrophilic group portion of the monolayer film becomes drawn onto the solid substrate. If the monolayer film is accumulated in an ideal manner, the hydrophobic group portion is exposed on the surface of the solid substrate.

In a case where the surface of the solid substrate does not become intimate with water, in opposition to the case where the substrate surface becomes intimate with water, the solid substrate is lowered below the water surface from above the monolayer film fabricated in advance on the water surface and is immersed into the water. Because the water surface becomes depressed slightly downward in accompaniment with the lowering of the solid substrate, the hydrophobic group portion of the monolayer film becomes drawn onto the solid substrate. If the monolayer film is accumulated in an ideal manner, the hydrophilic group portion is exposed on the surface of the solid substrate. Here, by then raising the solid substrate above the water surface as it is, another monolayer film can be accumulated in succession. By repeating this operation, an accumulated film of multiple layers can be obtained.

(Determination of Molecular Species and Concentration) Ultraviolet-Visible Spectroscopy Method

Two important information items can be obtained by the ultraviolet-visible spectroscopy method. One is an absorption wavelength ($\lambda_{max}$, nm). This contains energy information on electronic transition and provides information related to an actual chromophore that exhibits absorption. The other is a quantity called molar extinction coefficient ε, which is a quantity intrinsic to a molecule at a fixed wavelength and is an indicator of how easily an electronic transition occurs due to absorption of light. If the electronic transition occurs readily, the light is absorbed strongly and the value of ε is large. Oppositely, if the transition does not occur readily, the value of ε is small. A relationship between the molar extinction coefficient and a concentration (mol $L^{-1}$) of a chromophore is expressed by the Lambert-Beer law.

[Formula 1]

$$\log(I_o/I)=\varepsilon cl$$

The relationship can also be expressed as follows:

[Formula 2]

$$\varepsilon=A/cl$$

In the above, $I_o$=incident intensity, I=transmitted light intensity, ε=molar extinction coefficient, c=molar concentration, 1=optical path length of sample solution in units of cm, A=absorbance= $\log(I_o/I)$.

An ultraviolet-visible spectrometer records the absorbance directly onto a hard disk, which is a data recording medium. A sample cell is normally fabricated to have an optical path length of 1cm. In this case, ε is determined directly by the following formula:

[Formula 3]

$$\varepsilon=A/c$$

(Determination and Quantification of Chemical Bond States and Functional Groups of a Molecule) Infrared Spectroscopy Method

Vibration and rotation states of a molecule are excited by absorption of electromagnetic waves in an infrared region. This region lies at a longer wavelength side of the visible region. Such information related to the vibration and rotation of the molecule can be obtained directly from absorption in an infrared (IR) spectrum. A position of an absorption band in an IR spectrum can be indicated by an infrared wavelength $\lambda$ (in units of $\mu$ or $\mu$m). Characteristic absorption bands that are used in structural analysis of organic compound molecules lie in the following region.

[Formula 4]

$$\lambda=2.5 \text{ to } 15\mu m \; (1\mu m=10^{-3}mm=10^{4}Å)$$

In recent years, an inverse of the wavelength, that is, a wavenumber $\nu^{-}$ ($cm^{-1}$) is used. A numerical value ($cm^{-1}$) of $\nu^{-}$ indicates how many infrared waves are present per 1cm.

[Formula 5]

$$\text{Wavenumber } \nu^{-}=1/\lambda$$

By a relationship formula, the wavenumber ($cm^{-1}$) can be converted to wavelength ($\mu$m). That is,

[Formula 6]

$$\text{Wavenumber } \nu^{-} \; (cm^{-1})=10^{4}/\text{wavelength } \lambda \; (\mu m)$$

The wavenumber $\nu^{-}$ is proportional to a vibration number $\nu$ of an absorption line. It can thus be understood from the following relationship formula that the wavenumber is also proportional to a vibration energy $\Delta E$.

[Formula 7]

$$\lambda\nu=c$$

$$\nu=c/\lambda=c\nu^{-}$$

$$\Delta E=h\nu=hc/\lambda=hc\nu^{-}$$

$$\Delta E\sim\nu^{-}$$

In the above, c: speedof light ($3\times10^{10}cm\cdot s^{-1}$) , h: Planck's constant, v: vibration number (Hz or $s^{-1}$), $\lambda$: wavelength (cm), $\nu^{-}$: wavenumber ($cm^{-1}$).

Normally in an IR spectrum, a wavenumber region of 4000 to 400$cm^{-1}$ is used.

Many functional groups in organic compound molecules exhibit characteristic vibrations corresponding to absorption bands in a certain region in the IR spectrum. Molecular vibration occurs locally within a functional group and does not spread to other portions inside the molecule. Presence of a specific functional group can thus be determined by its absorption band.

Precise analysis is enabled by use of a tapping mode AFM of a scanning prove microscope to measure a surface structure of a fabricated thin film. With this measurement method, a piezoresonance vibrator is used to vibrate a cantilever, having a probe attached to a tip, at a frequency near a resonance frequency (approximately 50 to 500kHz) to perform scanning while lightly and intermittently contacting (tapping) a sample surface. A variation amount of an amplitude of the cantilever due to unevenness of the sample surface is detected using laser light. To keep the detected cantilever amplitude fixed, a Z-axis of a piezoscanner is moved vertically by feedback. At the same time, raster scanning in X- and Y-axis directions is performed, and drawing of a three-dimensional surface shape is enabled based on the X-, Y-, and Z-axis drives.

The method according to the present invention can be briefly summarized as follows. First, a fatty acid is prepared as a solidpowder sample and dissolved in chloroform as a solvent. This is spread on a water surface and is accumulated from above the water surface as a film onto a glass plate or a silica substrate by the LB method or other conventional method. The substrate with the film placed thereon is cleaned with distilled water and then dried in a nitrogen gas atmosphere. Next, in likewise manner, a lipid is prepared as a solid powder sample and dissolved in hexane as an organic solvent. Because the lipid is insoluble, dispersion by ultrasonic waves is performed, and in a short time before reaggregation occurs, the lipid is spread on a water surface and then accumulated by the LB method or other conventional method from above the water surface as a film onto the substrate, on which the fatty acid was accumulated. The state of the thin film is examined by identification of the accumulated substance and observation of the state by measurement of the ultraviolet-visible absorption spectrum and the infrared absorption spectrum and by scanning probe microscope measurement to determine fabrication conditions suited for the thin film that is targeted. If a dense, uniform bilayer filmof the lipid is constructed, a state enabling mixing and introduction of a membrane protein in the process of introducing the lipid as a reagent is achieved. The system is subsequently tested as a biomolecular element. With the present invention, film fabrication that suits the purpose is realized by combination with the fatty acid in the stage of constructing the bilayer film of the lipid.

Principal points of an implementation procedure are as follows:

1) Use an LB film fabricating apparatus and fill a water bath with distilled water.
2) Clean the water surface using a suction pump.
3) Set a float and immerse a cleaned substrate onto the water surface (Y type vertical dipping method).
4) Use a syringe to spread a predetermined reagent slowly on the water surface and leave alone for 20 minutes to wait for volatilization of hexane, which is the solvent.
5) Place a weight on the float and make organic molecules on the water surface aggregate due to surface pressure.
6) Start up a motor for performing raising/lowering of the substrate and begin film forming.
7) After the end of a predetermined number of times of accumulation onto the substrate, clean with distilled water, dry under a nitrogen gas atmosphere, and perform ultraviolet-visible absorption spectrum measurement and infrared absorption spectrum measurement to analyze light absorption characteristics.
8) Use a scanning probe microscope to perform surface structure analysis of the thin film.

Details of the above shall now be described.

Reference Example 1

As a first stage, DMPC (purchased from Nacalai Tesque Inc.) of solid powder form was first prepared as a sample, weighed out into a vial to achieve a concentration of 3.2mM at a volume of 5mL, hexane solvent was added to achieve a volume of 5mL, and the vial was subject to ultrasonic vibration by a table-top ultrasonic oscillator (NEOCLEANER-R, manufactured by Alex Corporation) to disperse the DMPC in the hexane that is a solvent. It was possible to determine by the naked eye that a uniformly dispersed state was achieved. Using a syringe, 60μl of the solution was batched off rapidly and spread slowly on a water surface, under which a silica substrate of 10mm×15mm×1mm size was immersed vertically in advance to a depth of 10mm in distilled water accumulated and left still in a water bath of an LB method fabricating apparatus designed and fabricated by the inventor, and then left still for 20 minutes for volatilization of the organic solvent and formation of the film.

In performing the molecular film fabricating experiment on the water surface by the LB method, the mass of the weight was considered as follows. By using the weight to make small an area, surrounded by the float and a Teflon (registered trademark) frame, it is possible to align molecules, floating in an unordered manner, to an ordered array in

which an area occupied per molecule is small. If the mass of the weight is too large, the pressure applied to the water surface becomes high and the molecular layer on the water phase may overlap and no longer be a monolayer or the molecular layer spread on the water surface may become destroyed in itself. The mass of the weight is thus also an important value in the LB film fabricating experiment. The value of the surface pressure of the film varies according to the mass of the weight. A shape, of the apparatus, characteristics of the molecule, and various other factors are involved. The monolayer spread on the surface of the water phase becomes aligned in an orderly manner at the molecular level by the surface pressure in accompaniment with the decrease, by the weight, of the area surrounded by the Teflon (registered trademark) frame and the float. It is considered that by increasing the mass of the weight, the surface pressure applied to the monolayer increases and the area surrounded by the Teflon (registered trademark) frame and the float becomes a constant value. It is predicted that when the mass of the weight is increased further, the monolayer spread on the water surface becomes unable to bear the surface pressure and becomes overlapped or destroyed. It is considered that at that instant, the surface pressure applied on the monolayer is released and the area surrounded by the Teflon (registered trademark) frame and the float decreases suddenly. The weight immediately before this state is considered to achieve a state where the monolayer is aligned in a most orderly manner and can be said to be suited for LB film fabrication. In a preliminary experiment stage, up to a load of 0.1 to 0.14g, the molecules spread on the water surface, became assembled by the decrease of the area surrounded by the Teflon (registered trademark) frame and the float, and the occupied area decreased. With a load of 0.15 to 0.18g, a state where a monolayer, in which molecules are aligned in an orderly manner, can be maintained is achieved and this was considered to be the load suitable for the LB film fabricating experiment. With a load of no less than 0.19g, the monolayer that was spread on the water surface could not bear the surface pressure, and it was predicted that at higher load values, the monolayer becomes destroyed.

In considering the area occupied per molecule, it was also considered that in fabricating a monolayer inside a restricted range, there is a limit to the number of molecules that can be spread on the surface. With the water bath of the LB film fabricating apparatus used, the inner side of the frame is approximately 187.2 [cm], and when the float is floated, the range of spreading of molecules is approximately 150 [cm]. Theoretically, when the load is 0.15 to 0.18g, if it is assumed that a monolayer, in which the molecules are aligned in an orderly manner, is spread on the water surface, a relationship of the area occupied per molecule can be expressed by the following formula:

[Formula 8]

$$A=S/m\times N_A$$

In the above, m=amount of spread molecules [mol], S=area of the water surface, A=area occupied per molecule [$Å^2$], and $N_A$=Avogadro's number [$6.02\times10^{23}$].

When the area value of the water surface and actual numerical values in the case of a weight of 0.15g are substituted in the above formula, A=38.9 [Å]. Ikonen et al reports the area occupied per molecule in a uniformly compressed state to be 50$Å^2$ in the case of DMPC molecules and it can be said that the numerical value obtained in the present experiment matches this substantially.

The DMPC film, thus formed by the hydrophobic interaction forces among molecules, the interface adsorption with water molecules upon spreading the film of DMPC molecules on the water surface and applying an appropriate load of 0.15g on the float, was drawn up at a slow speed of 0.01mm/sec. When the substrate was raised completely into the gas phase, the substrate was lowered at a speed of 0.01mm/sec, stopped at a depth in water of 10mm, and then raised by the same operation as described above. An LB film of DMPC was thus fabricated. This was fabricated as a three-layer LB film. An ultraviolet-visible absorption spectrum of the film was examined using an ultraviolet-visible spectrometric measuring apparatus (UV-1200, manufactured by Shimadzu Corp.). The result is shown as "(1) 3 layers" in FIG. 1. An infrared absorption spectrum was examined using a Fourier transform infrared spectrophotometer (FTIR-8400S, manufactured by Shimadzu Corp.). The result is shown as "(1) 3 layers" in FIG. 2. Also, a surface structure was examined with a scanning probe microscope (Nano Scope IIIa Tapping mode AFM, manufactured by Digital Instruments Inc.). The results are shown in FIGS. 3 (1) and 3 (4). A five-layer LB film and a seven-layer LB film were fabricated by additional lowering and raising of the substrate in a likewise manner, and the ultraviolet-visible absorption spectra, the infrared absorption spectra, and the surface structures of these films were measured in a likewise manner. The respective results are shown as "(2) 5 layers" in FIG. 1, "(2) 5 layers" in FIG. 2, and in FIGS. 3(2) and 3(5) and as " (3) 7 layers" in FIG. 1, " (3) 7 layers" in FIG. 2, and in FIGS. 3 (3) and 3 (6). In the ultraviolet-visible absorption spectra of " (1) 3 layers," " (2) 5 layers," and "(3) 7 layers" of FIG. 1, light absorption characteristics of DMPC are seen near 260nm and 330nm, and light absorption values increased with the increase of number of layers. With the infrared absorption spectra of " (1) 3 layers," "(2) 5 layers," and "(3) 7 layers" of FIG. 2, infrared absorption by DMPC is seen over a wide wavenumber range and absorption values increased with the increase of number of layers. This can be seen clearly, for example,

near 1300cm$^{-1}$. However, from the measurement results of the surface structure of FIG. 3, it became clear that a uniform film may not be formed necessarily when the DMPC film is accumulated directly onto the silica substrate by the LB method. As a result of conducting an experiment of increasing the number of accumulated layers (nine layers, eleven layers, and thirteen layers) and measuring the two types of spectra, the light absorption value did not increase above that of the seven-layer film. It was thus possible to draw a layer number variation model of the accumulated state as shown in FIG. 4. That is, although the DMPC 2 cannot become attached to the silica substrate 1 uniformly, it becomes attached partially. With this model, although the DMPC molecules can be accumulated on the attached DMPC molecules, the limit is approximately seven layers.

Example 1

Next, as a second stage, an experiment of not accumulating DMPC directly on a silica substrate but of accumulating the fatty acid; stearic acid, and accumulating the DMPC above the stearic acid was conducted. First, stearic acid (purchased from Nacalai Tesque Inc.) was prepared as a sample, weighed out into a vial to achieve a concentration of 3.2mM at a volume of 5mL, chloroform solvent was added to achieve a volume of 5mL, and a three-layer film was fabricated in the same manner as described above. Thereafter, a two-layer film was fabricated using a DMPC film spread on a separately prepared water bath. An LB film of three layers of stearic acid and two layers of DMPC was thereby fabricated. The infrared absorption spectrum and the surface structure of this film were examined in the same manner as described above. The results are shown in FIGS. 5 and 6. (1) in FIG. 5 is an infrared absorption spectrum of a reference LB film of three layers of stearic acid, and (2) is an infrared absorption spectrum of the LB film of three layers of stearic acid + two layers of DMPC. These show that the infrared absorption characteristics of DMPC, such as can be seen in FIG. 2, are observed more clearly as a difference. The values were also large. FIG. 6(2) is an AFM image as observed from an angle of 90 degrees above the film, and what appears as a black hole is a recessed, flat portion, and FIG. 6(3) is an AFM image obtained by measurement from an angle of 45 degrees upon magnification. Cross-sectional analysis results are shown in FIG. 6 (4). From these results, it became clear that a film, dotted with recessed flat portions, each of circular shape of approximately 200nm, is formed and that flat portions of the film that are not recesses (the portions that appear white in FIG. 6(2) and (3)) are a uniformly dense DMPC film. It also became clear from the results of cross-sectional analysis (FIG. 6(4)) that there is a fine unevenness of a step height corresponding to just a single molecule of DMPC (approximately 3.3nm) within the smooth film that appears white and that there is a step difference corresponding to approximately two molecules between the smooth flat portion and the recessed flat portion. FIG. 7 shows a film structure model drawn based on the above. This model expresses that a smooth DMPC film (circular step) is formed with in an entirety dotted with recessed smooth portions, with a diameter of approximately 200nm each and indicated by 5 in FIG. 7. Although such a characteristic partial structure appears to be non-usable at first glance, it has a possibility of functioning as a container for a macromolecule or an aggregate thereof. It also became clear from cross-sectional analysis that the smooth film portion that appears white has a smoothness of 0.5 to 1nm, and when a membrane protein is to be introduced, it is to be contained in this smooth portion.

In FIG. 7, 1 is the silica substrate, 2 is DMPC, and 3 is stearic acid.

Example 2

Next, as a third stage, an experiment of not accumulating DMPC directly on a silica substrate but of accumulating the fatty acid, retinoic acid, and accumulating DMPC above the retinoic acid was conducted. First, retinoic acid (purchased from Sigma Chemical Co.; liquid reagent placed in an ampoule) was prepared as a sample, weighed out into a vial to achieve a concentration of 3.2mM at a volume of 5mL, chloroform solvent was added to achieve a volume of 5mL, and a three-layer film was fabricated in the same manner as described above. Thereafter, a two-layer film was fabricated using a DMPC film spread on a separately prepared water bath. An LB film of three layers of retinoic acid and two layers of DMPC was thereby fabricated. The infrared absorption spectrum and the surface structure of this film were examined in the same manner as described above. The results are shown in FIGS. 8 and 9. (1) in FIG. 8 is an infrared absorption spectrum of a reference LB film of three layers of retinoic acid, and (2) is an infrared absorption spectrum of the LB film of three layers of retinoic acid + two layers of DMPC. These show that the infrared absorption characteristics of DMPC, such as can be seen in FIG. 2, are observed more clearly as a difference. The values were also large. The values are approximately five times in comparison with the data for stearic acid. FIG. 9(2) is an AFM image as observed from an angle of 90 degrees above the film, and characteristic, recessed flat portions were not seen as in the case of stearic acid, and the entirety of the film was constituted of a uniformly uneven surface. FIG. 9(3) is an AFM image obtained by measurement from an angle of 48 degrees upon magnification. Cross-sectional analysis results are shown in FIG. 9(4). From these results, it became clear that the film is constituted of a collection of uniform, curved protruding surfaces of approximately 200nm. It also became clear from the results of cross-sectional analysis (FIG. 9(4)) that the uniform film has an unevenness close to a step height corresponding to two DMPC molecules. FIG. 10 shows a film structure model

drawn based on the above. This model expresses that a uniform, dense DMPC film (lipid curved surface) is formed in an entirety in which gradually protruding curved surface portions, with a maximum height difference of several nm and width of approximately 200nmas indicated by 10 (6), are assembled. In this case, when a membrane protein is to be introduced, there is a possibility that it can be contained in any portion of the uniform film.

In FIG. 10, 1 is the silica substrate, 2 is DMPC, and 3 is retinoic acid.

Industrial Applicability

What was discovered by the stage-wise experimental researchdescribedabove is that a lipidbilayer film, fabricated above a fatty acid film by a comparatively simple LB film forming method of transferring organic molecules onto a substrate without use of chemical bonds is formed, in accordance with the combination of the molecules laminated, as a dense bilayer film, having characteristic structures of flat surfaces and circular steps formed by generation of curved lipid surfaces, or as a dense, uniform film.

Specifically, the DMPC/stearic acid film and the DMPC/retinoic acid film were fabricated. The thin film obtained in the case of the DMPC/stearic acid film has extremely smooth portions and circular steps of a diameter of approximately 200nm, and the thin film obtained in the case of the DMPC/retinoic acid film is a flexible structure that is uniformly dense in entirety and has gradually curved surface shapes. Both films can be used as supports for a membrane protein as a biomolecular element.

A most important point is that the films are systems close in concept to a biological film system. The two conditions of realizing the fine functions of a receptor protein or an ion transport protein and being an artificial thin film are met. The films are usable as bases for molecular device construction and manufacture. It is considered that a lipid/fatty acid laminated film can be designed to suit size and interface adsorption characteristics of a molecule to be introduced or an aggregate system thereof.

As mentioned above, in terms of industrial applicability, it is anticipated that the films can be used for example as supports for a membrane protein used in parts that are basic units of an advanced function device, such as a service robot, medical apparatus (for example, an olfactory sensor device for urine, sweat or other secreted substance from a human body or a chemical substance).

BRIEF DESCRIPTION OF THE DRAWINGS

[FIG. 1] is a diagram of ultraviolet-visible absorption spectra of DMPC directly accumulated on a silica substrate for cases where a number of layers of a DMPC accumulation state is three (1), five (2), and seven (3).

[FIG. 2] is a diagram of infrared absorption spectra of DMPC directly accumulated on the silica substrate for cases where the number of layers of the DMPC accumulation state is three (1), five (2), and seven (3).

[FIG. 3] shows AFM images of surface structures of DMPC directly accumulated on the silica substrate as measured by a scanning probe microscope with FIGS. 3 (1) and (4) being AFM images for the case of three layers, FIGS. 3 (2) and (5) being AFM images for the case of five layers, and FIGS. 3 (3) and (6) being AFM images for the case of seven layers.

[FIG. 4] shows diagrams of models of layer number variations of DMPC accumulation states achieved by direct accumulation onto the silica substrate with FIG. 4(1) being a diagram of a model for three layers, FIG. 4 (2) being a diagram of a model for five layers, and FIG. 4(3) being a diagram of a model for seven layers.

[FIG. 5] shows infrared absorption spectra with (1) being that of just three layers of stearic acid and (2) being that of three layers of stearic acid + two layers of DMPC and is thus that of a thin film fabricated by accumulating DMPC after accumulation of stearic acid on a silica substrate.

[FIG. 6] shows AFM images measured by the scanning probe microscope of surface structures of the thin film, fabricated by accumulating DMPC after accumulation of stearic acid on the silica substrate, with FIG. 6 (1) being an AFM image of 10μm×10μm scale as viewed from 45 degrees above, FIG. 6(2) being an AFM image of 10μm×10μm scale as viewed from 90 degrees above, FIG. 6(3) being an AFM image of 500nm×500nm scale as viewed from 45 degrees above, and FIG. 6 (4) showing an AFM image of 500nm× 500nm scale as viewed from 90 degrees above and results of cross-sectional analysis of a structural portion of a step, including an end of a recessed flat surface that is noted in the description.

[FIG. 7] is a diagram of a structural model of the thin film fabricated by accumulating DMPC after accumulation of stearic acid on the silica substrate.

[FIG. 8] shows infrared absorption spectra with (1) being that of just three layers of retinoic acid and (2) being that of three layers of retinoic acid + two layers of DMPC and is thus that of a thin film fabricated by accumulating DMPC after accumulation of retinoic acid on a silica substrate.

[FIG. 9] shows AFM images measured by the scanning probe microscope of surface structures of the thin film, fabricated by accumulating DMPC after accumulation of retinoic acid on the silica substrate, with FIG. 9(1) being an AFM image of 10μm×10μm scale as viewed from 45 degrees above, FIG. 9 (2) being an AFM image of 10μm×10μm scale as viewed from 90 degrees above, FIG. 9 (3) being an AFM image of 500nmx500nm scale as viewed from 45 degrees above, and FIG. 9(4) showing an AFM image of 500nm× 500nm scale as viewed from 90 degrees above and results of cross-sectional analysis of a structural portion, including an uneven curved surface that is noted in the description.

[FIG. 10] is a diagram of a structural model of the thin film fabricated by accumulating DMPC after accumulation of retinoic acid on the silica substrate.

Description of the Reference Numerals

1 Silica substrate ($SiO_2$ crystal plate)

2 DMPC

3 Stearic acid

4 Retinoic acid

5 DMPC film (circular step)

6 DMPC film (lipid curved surface)

## Claims

**1.** A thin film comprising a laminate of molecular thin films of a fatty acid and lipid; wherein the laminate of molecular thin films comprises a first type layer of a fatty acid and a second type layer of a lipid; and the first type layer is formed on a substrate and the second type layer is joined onto the first type layer

**2.** The thin film according to Claim 1, wherein the fatty acid is a fatty acid having an ionone ring or a combination of a fatty acid having an ionone ring and an alkane acid.

**3.** The thin film according to Claim 2, wherein the fatty acid having an ionone ring is retinoic acid.

**4.** The thin film according to Claim 1, wherein the lipid is phospholipid.

**5.** The thin film according to Claim 4, wherein the phospholipid is dimyristoylphosphatidylcholine or dipalmitoylphosphatidylcholine.

**6.** The thin film according to any one of Claims 1 - 5, wherein the first layer is three-layer molecular thin films of a fatty acid.

**7.** The thin film according to any one of Claims 1 - 5, wherein the second layer is two-layer molecular thin films of a lipid.

**8.** The thin film according to Claim 1 - 7, wherein the surface of the thin film is constituted of a collection of uniform, curved protruding surfaces of approximately 200 nm.

**9.** The thin film according to Claim 7, wherein a membrane protein is stabilized and expresses a function in the two-layer molecular thin films of a lipid.

**10.** A method for producing an organic thin film, wherein a first layer of fatty acid is formed on a substrate by a vertical dipping method and a second type of layer of a lipid suspended in a solvent is dispersed by ultrasonic wave application is joined from above to the first layer of fatty acid.

**11.** The method for producing an organic thin film or a laminated molecular film according to Claim 10, wherein the

organic thin film has a basic configuration of having the lipid molecules on an uppermost stage and the fatty acid below.

12. The method for producing an organic thin film according to Claim 11, wherein an organic solvent is used as the solvent in which the lipid is dispersed before the organic thin film joining process.

13. The method for producing an organic thin film according to Claim 12, wherein an organic solvent with a boiling point in a range of 40°C to 70°C is used as the organic solvent.

14. The method for producing an organic thin film according to Claim 13, wherein hexane is used as the organic solvent.

15. The method for producing an organic thin film according to Claim 10, wherein, to introduce a membrane protein or other molecule in the organic thin film, the membrane protein or other molecule is mixed in a phospholipid suspended in a organic solvent to obtain the organic thin film having the lipid molecules and the membrane protein or other molecule at the uppermost stage and the fatty acid below.

16. The method for producing an organic molecular thin film according to Claim 10, wherein in the vertical dipping method, the organic thin film is formed accumulatingly on the substrate by a Langmuir-Blodgett method.

## Patentansprüche

1. Dünnfilm, der ein Laminat von molekularen Dünnfilmen aus einer Fettsäure und Lipid umfasst; wobei das Laminat von molekularen Dünnfilmen eine erste Art von Schicht aus einer Fettsäure und eine zweite Art von Schicht aus einem Lipid umfasst; und wobei die erste Art von Schicht auf einem Substrat gebildet ist und die zweite Art von Schicht auf die erste Art von Schicht angefügt ist.

2. Dünnfilm nach Anspruch 1, wobei die Fettsäure eine Fettsäure mit einem Ionon-Ring oder eine Kombination aus einer Fettsäure mit einem Ionon-Ring und einer Alkansäure ist.

3. Dünnfilm nach Anspruch 2, wobei die Fettsäure mit einem Ionon-Ring Retinsäure ist.

4. Dünnfilm nach Anspruch 1, wobei das Lipid Phospholipid ist.

5. Dünnfilm nach Anspruch 4, wobei das Phospholipid Dimyristoylphosphatidylcholin oder Dipalmitoylphosphatidylcholin ist.

6. Dünnfilm nach einem der Ansprüche 1 - 5, wobei die erste Schicht Drei-Schichtmolekulardünnfilme aus einer Fettsäure ist.

7. Dünnfilm nach einem der Ansprüche 1 - 5, wobei die zweite Schicht Zwei-Schichtmolekulardünnfilme aus einem Lipid ist.

8. Dünnfilm nach Anspruch 1 - 7, wobei die Oberfläche des Dünnfilms aus einer Zusammenstellung von einheitlichen, gebogen hervorstehenden Oberflächen von ungefähr 200 nm zusammengesetzt ist.

9. Dünnfilm nach Anspruch 7, wobei ein Membranprotein stabilisiert ist und eine Funktion in den Zwei-Schichtmolekulardünnfilmen aus einem Lipid ausdrückt.

10. Verfahren zur Herstellung eines organischen Dünnfilms, wobei eine erste Schicht aus Fettsäure auf einem Substrat durch ein vertikales Tauchverfahren gebildet wird und eine zweite Art von Schicht aus einem in einem Lösungsmittel suspendierten Lipid durch Ultraschallwellenbehandlung dispergiert wird und von oben zu der ersten Schicht aus Fettsäure angefügt wird.

11. Verfahren zur Herstellung eines organischen Dünnfilms oder eines laminierten molekularen Films nach Anspruch 10, wobei der organische Dünnfilm eine Grundkonfiguration aufweist, die die Lipidmoleküle an dem obersten Abschnitt und die Fettsäure darunter aufweist.

12. Verfahren zur Herstellung eines organischen Dünnfilms nach Anspruch 11, wobei ein organisches Lösungsmittel

als das Lösungsmittel verwendet wird, in welchem das Lipid vor dem organischen Dünnfilmanfügungsprozess dispergiert wird.

13. Verfahren zur Herstellung eines organischen Dünnfilms nach Anspruch 12, wobei ein organisches Lösungsmittel mit einen Siedepunkt in einem Bereich von 40° C bis 70°C als das organische Lösungsmittel verwendet wird.

14. Verfahren zur Herstellung eines organischen Dünnfilms nach Anspruch 13, wobei Hexan als das organische Lösungsmittel verwendet wird.

15. Verfahren zur Herstellung eines organischen Dünnfilms nach Anspruch 10, wobei, um ein Membranprotein oder ein anderes Molekül in den organischen Dünnfilm einzuführen, das Membranprotein oder das andere Molekül in ein Phospholipid gemischt wird, das in einem organischen Lösungsmittel suspendiert ist, um den organischen Dünnfilm zu erhalten, der die Lipidmoleküle und das Membranprotein oder das andere Molekül an dem obersten Abschnitt und die Fettsäure darunter aufweist.

16. Verfahren zur Herstellung eines organischen molekularen Dünnfilms nach Anspruch 10, wobei bei dem vertikalen Tauchverfahren der organische Dünnfilm auf dem Substrat durch an Langmuir-Blodgett-Verfahren akkumulativ gebildet wird.

## Revendications

1. Film mince comprenant un stratifié de films minces moléculaires d'un acide gras et d'un lipide ; dans lequel le stratifié de films minces moléculaires comprend une couche d'un premier type d'un acide gras et une couche d'un deuxième type d'un lipide ; et la couche d'un premier type est formée sur un substrat et la couche d'un deuxième type est jointe sur la couche du premier type.

2. Film mince selon la revendication 1, dans lequel l'acide gras est un acide gras ayant un noyau ionone ou une combinaison d'un acide gras ayant un noyau ionone et d'un acide alcane.

3. Film mince selon la revendication 2, dans lequel l'acide gras ayant un noyau ionone est l'acide rétinoïque.

4. Film mince selon la revendication 1, dans lequel le lipide est un phospholipide.

5. Film mince selon la revendication 4, dans lequel le phospholipide est la dimyristoylphosphatidylcholine ou la dipalmitoylphosphatidylcholine.

6. Film mince selon l'une quelconque des revendications 1 à 5, dans lequel la première couche est des films minces moléculaires à trois couches d'un acide gras.

7. Film mince selon l'une quelconque des revendications 1 à 5, dans lequel la deuxième couche est des films minces moléculaires à deux couches d'un lipide.

8. Film mince selon la revendication 1 à 7, dans lequel la surface du film mince est constituée d'une collection de surfaces saillantes courbes, uniformes, d'environ 200 nm.

9. Film mince selon la revendication 7, dans lequel une protéine membranaire est stabilisée et exprime une fonction dans les films minces moléculaires à deux couches d'un lipide.

10. Procédé de production d'un film mince organique, dans lequel une première couche d'acide gras est formée sur un substrat par un procédé d'immersion verticale et un deuxième type de couche d'un lipide en suspension dans un solvant est dispersé par l'application d'ondes ultrasonores est joint par le haut à la première couche d'acide gras.

11. Procédé de production d'un film mince organique ou d'un film moléculaire stratifié selon la revendication 10, dans lequel le film mince organique a une configuration de base consistant à avoir les molécules de lipide sur un étage supérieur et l'acide gras en-dessous.

12. Procédé de production d'un film mince organique selon la revendication 11, dans lequel un solvant organique est

utilisé comme solvant dans lequel le lipide est dispersé avant que le processus d'assemblage du film mince organique.

**13.** Procédé de production d'un film mince organique selon la revendication 12, dans lequel un solvant organique avec un point d'ébullition dans une gamme de 40°C à 70°C est utilisé comme solvant organique.

**14.** Procédé de production d'un film mince organique selon la revendication 13, dans lequel l'hexane est utilisé comme solvant organique.

**15.** Procédé de production d'un film mince organique selon la revendication 10, dans lequel, pour introduire une protéine membranaire ou une autre molécule dans le film mince organique, la protéine membranaire ou l'autre molécule est mélangée dans un phospholipide en suspension dans un solvant organique pour obtenir le film mince organique ayant les molécules de lipide et la protéine membranaire ou une autre molécule à l'étage supérieur et l'acide gras en-dessous.

**16.** Procédé de production d'un film mince moléculaire organique selon la revendication 10, dans lequel dans le procédé d'immersion verticale, le film mince organique est formé de façon cumulative sur le substrat par une méthode de Langmuir-Blodgett.

FIG.1

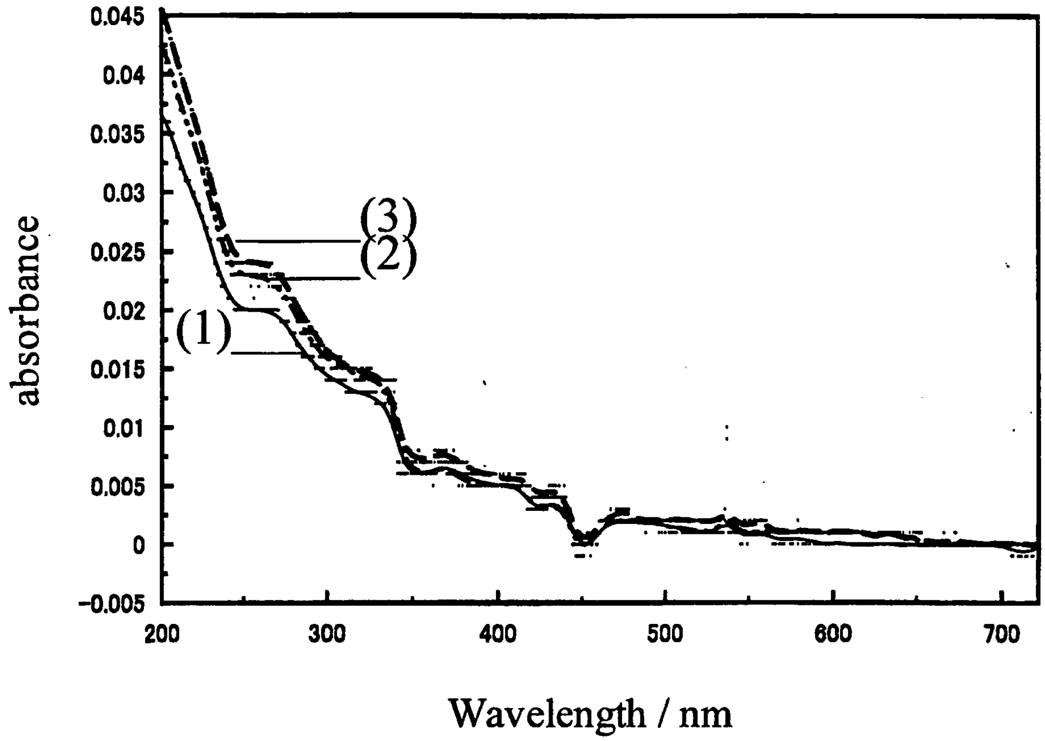
0.045
0.04
0.035
0.03
0.025
0.02
0.015
0.01
0.005
0
-0.005
absorbance
(3)
(2)
(1)
200
300
400
500
600
700
Wavelength / nm

FIG.2

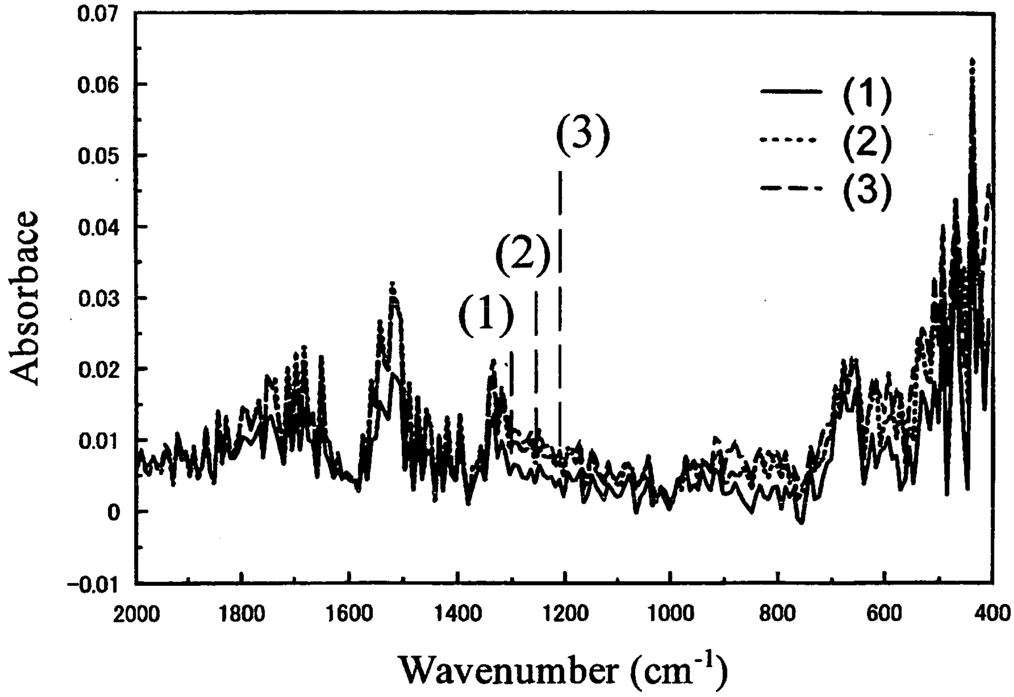
0.07
0.06
0.05
0.04
0.03
0.02
0.01
0
-0.01
Absorbace
(1)
(2)
(3)
(1)
(2)
(3)
2000
1800
1600
1400
1200
1000
800
600
400
Wavenumber (cm-1)

FIG.3

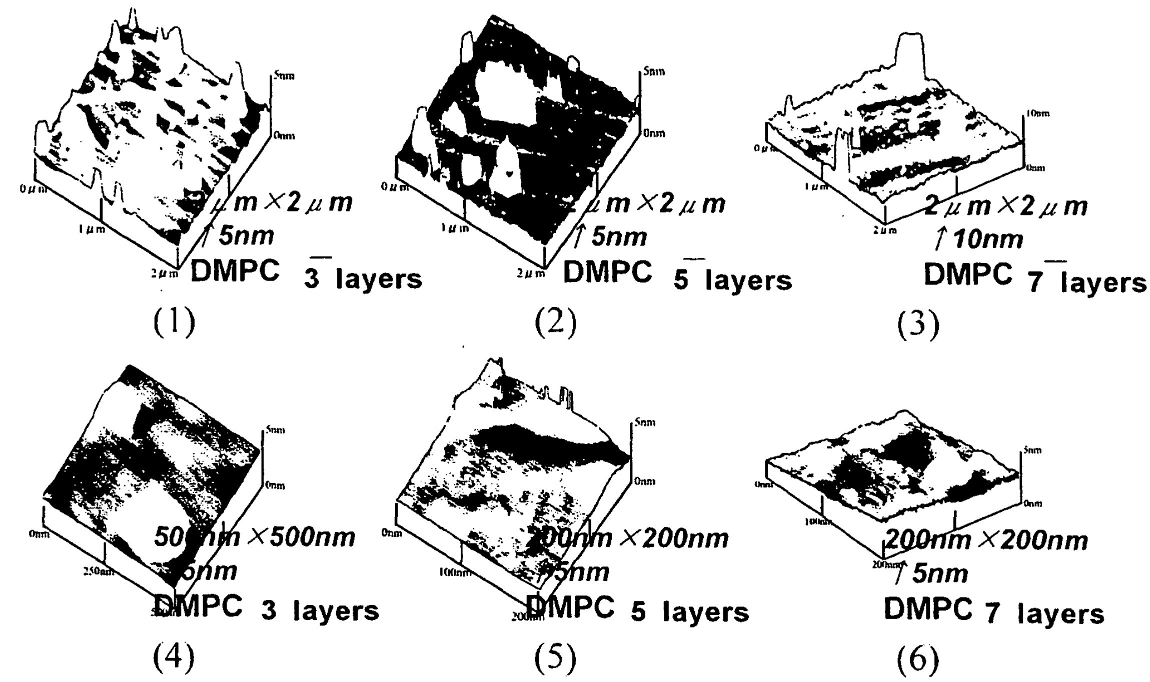
μm×2μm
↑5nm
DMPC 3 layers
(1)
μm×2μm
↑5nm
DMPC 5 layers
(2)
2μm×2μm
↑10nm
DMPC 7 layers
(3)
nm×500nm
DMPC 3 layers
(4)
nm×200nm
MPC 5 layers
(5)
200nm×200nm
↑5nm
DMPC 7 layers
(6)

FIG.4

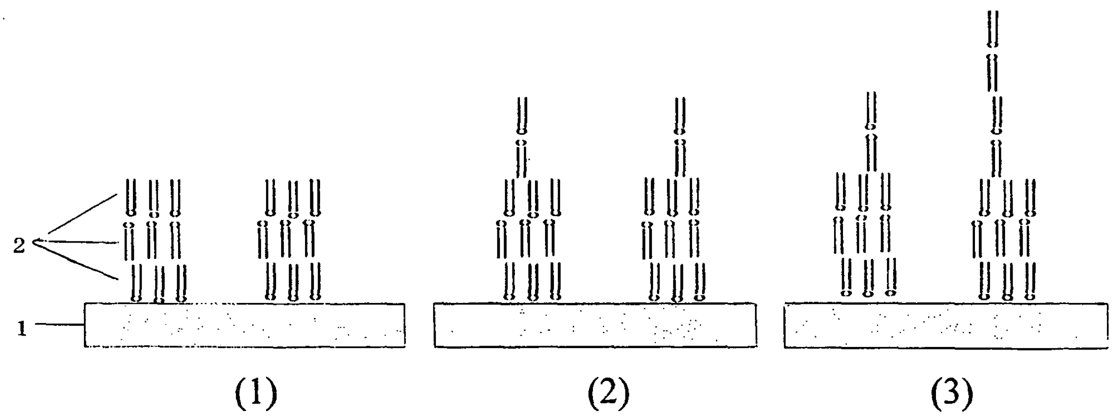
2
1
(1)
(2)
(3)

FIG.5

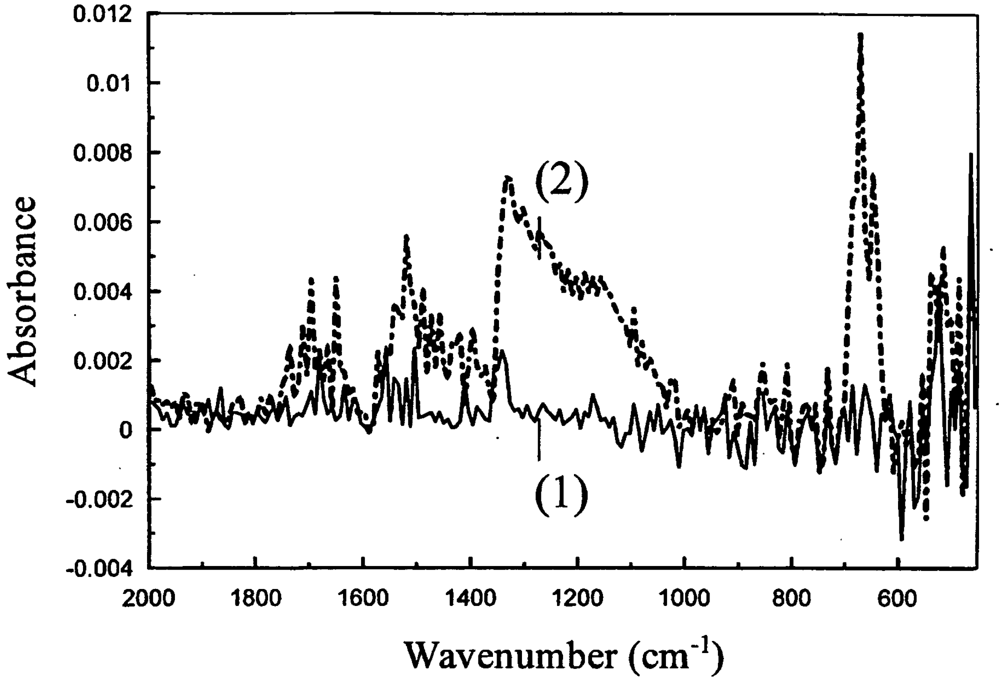
0.012
0.01
0.008
0.006
0.004
0.002
0
-0.002
-0.004
Absorbance
(2)
(1)
2000
1800
1600
1400
1200
1000
800
600
Wavenumber ($cm^{-1}$)

FIG.6

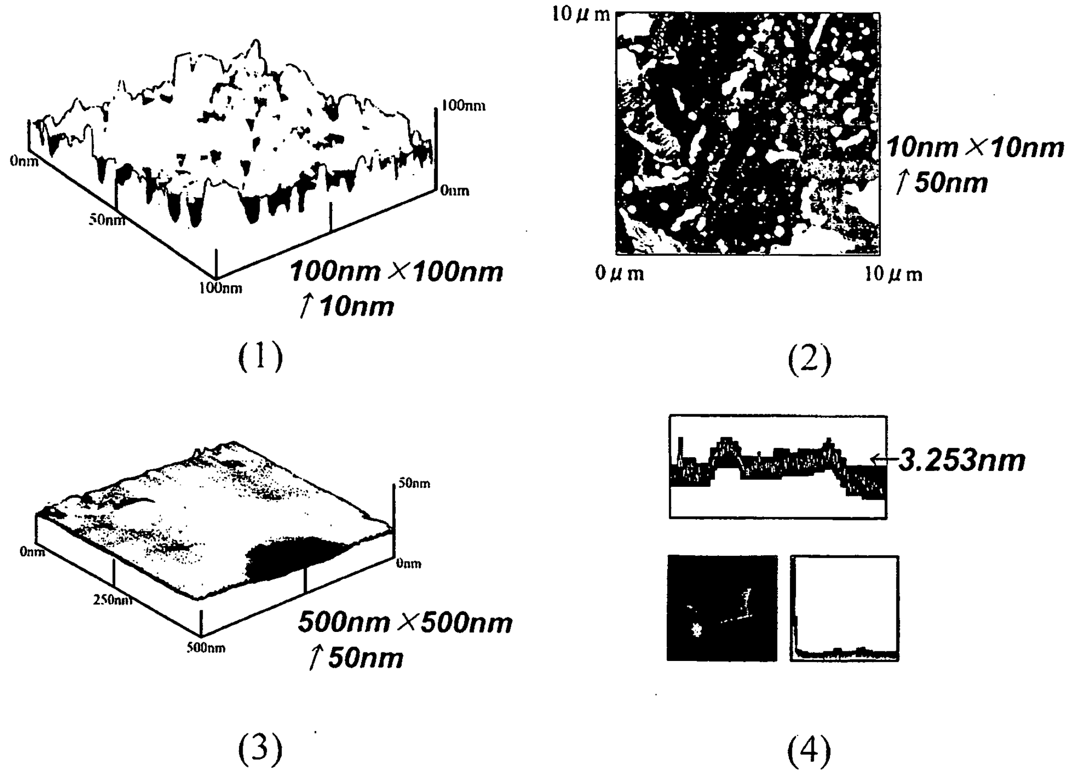
100nm
0nm
0nm
50nm
100nm
100nm × 100nm
↑10nm
(1)
10 μm
0 μm
10 μm
10nm × 10nm
↑50nm
(2)
50nm
0nm
0nm
250nm
500nm
500nm × 500nm
↑50nm
(3)
3.253nm
(4)

FIG.7

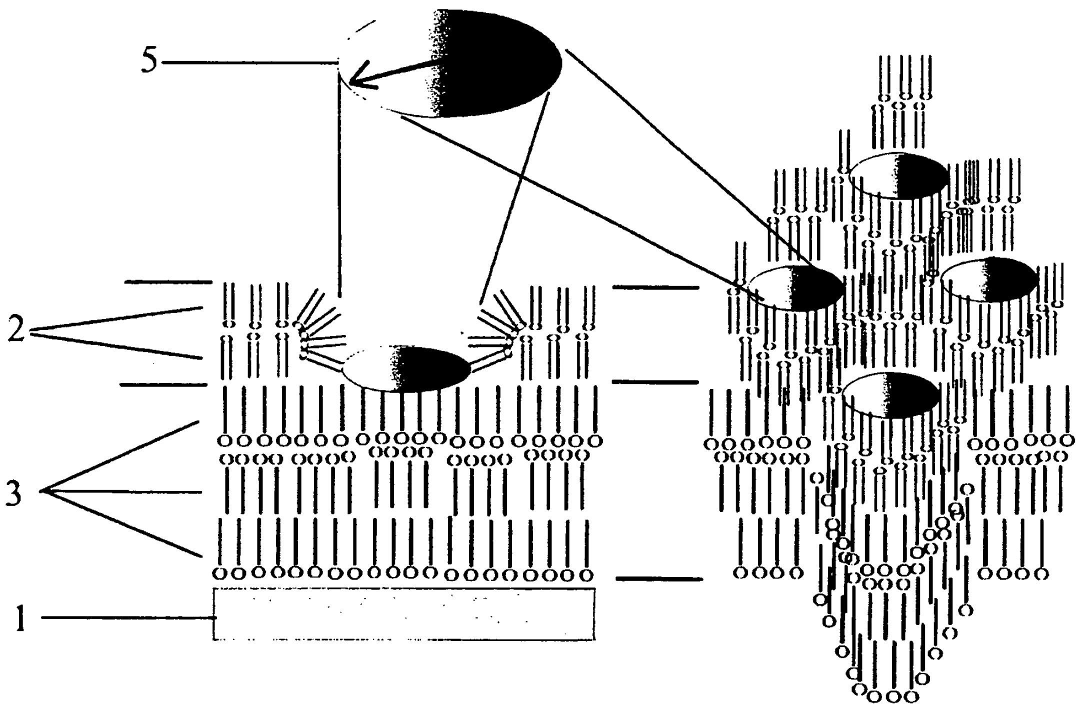
5
2
3
1

FIG.8

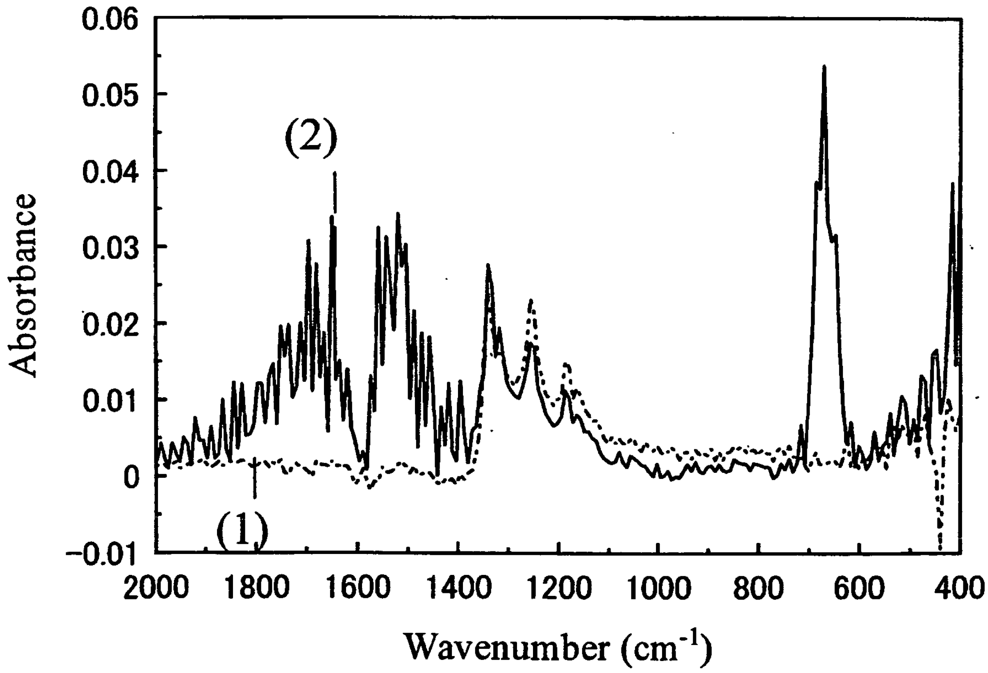
0.06
0.05
0.04
0.03
0.02
0.01
0
-0.01
Absorbance
(2)
(1)
2000
1800
1600
1400
1200
1000
800
600
400
Wavenumber (cm-1)

FIG.9

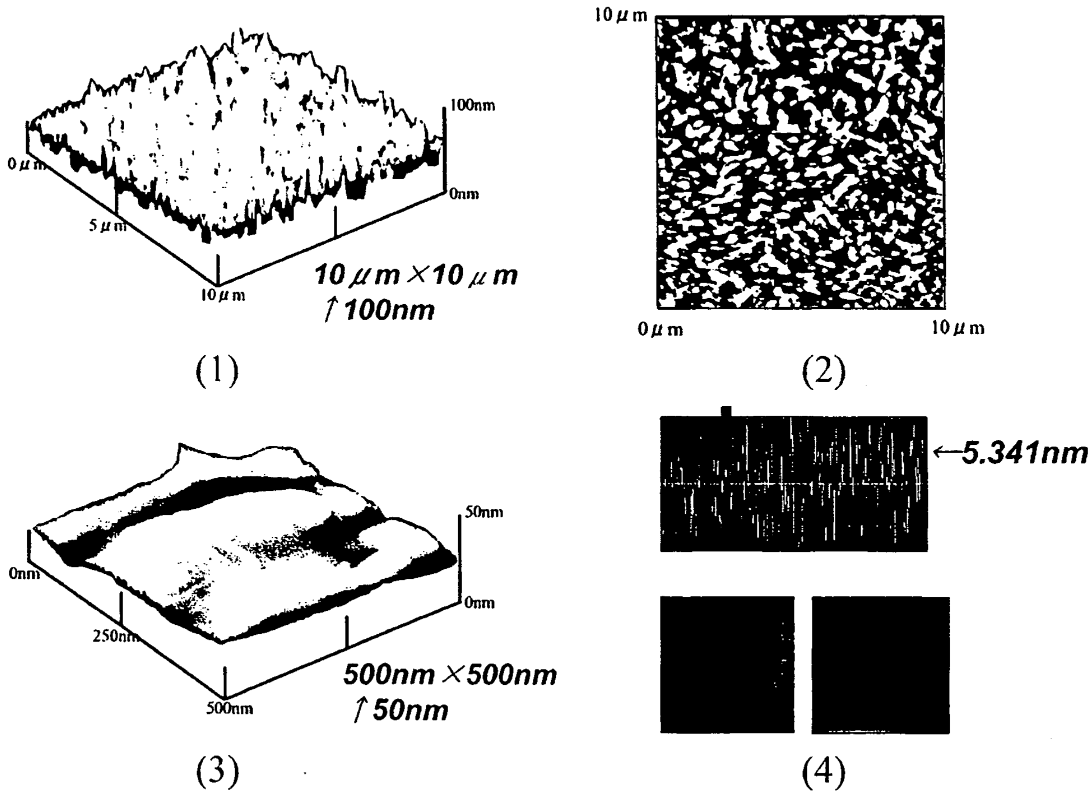
10 μm × 10 μm
↑100nm
100nm
0nm
0μm
5μm
10μm
(1)
10μm
0μm
10μm
(2)
500nm × 500nm
↑50nm
50nm
0nm
250nm
500nm
(3)
←5.341nm
(4)

FIG.10

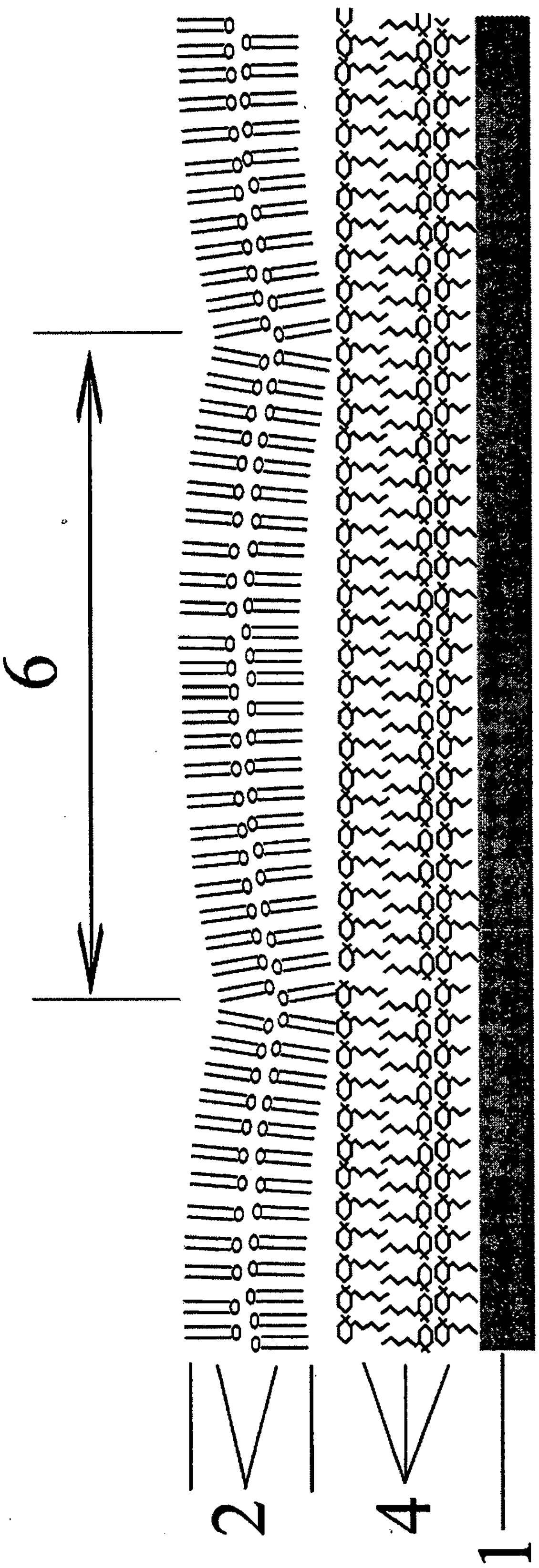

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- JP 2005245331 A


### Non-patent literature cited in the description


- **Marjo Ikonen ; Jouko Peltonen ; Elina Vuorimaa ; Helge Lemmetyinen.** Study of photocycle and spectral properties of bacteriorhodopsin in Langmuir-Blodgett films. *Thin Solid Films,* 1992, vol. 213, 277-284
- **HOWARTH VA ; PETTY MC ; ANCELIN H ; YARWOOD J.** structural characterisation of phospholipid Langmuir-Blodgett multilayers containing valinamycin. *VIBRATIONAL SPECTROSCOPY,* 1990, vol. 1, 29-33
- **BRUCKNER-LEA C ; PETELENZ D ; JANATA J.** Use of Poly(octadec-1-ene-maleic anhydride) for interfacing bilayer membranes to solid supports in sensor applications. *MIKROCHEMICA ACTA,* 1990, vol. 1, 169-185